# EUROPEAN PATENT SPECIFICATION

(11) **EP 4 009 782 B1**
(45) Date of publication and mention of the grant of the patent: **05.03.2025**
(21) Application number: 20745108.9
(22) Date of filing: 10.07.2020
(51) Int. Cl.: A01K 67/033

(54) **INSECT REARING BOX**
INSEKTENZUCHTKASTEN
BOÎTE D'ÉLEVAGE D'INSECTES

(30) Priority: 06.08.2019 EP 19382692
(43) Date of publication of application: 15.06.2022
(73) Proprietor: Alternative Gene Expression, S.L., 28760 Tres Cantos - Madrid (ES)
(72) Inventor: MARTÍNEZ ESCRIBANO, José Ángel, 28223 Pozuelo de Alarcón - Madrid (ES); CID FERNÁNDEZ, Miguel, 28223 Pozuelo de Alarcón - Madrid (ES); REYTOR SAAVEDRA, Edel, 28223 Pozuelo de Alarcón - Madrid (ES); ALVARADO FRADUA, Carmen, 28223 Pozuelo de Alarcón - Madrid (ES); MORENO DALTON, Romy, 28223 Pozuelo de Alarcón - Madrid (ES)
(74) Representative: Hoffmann Eitle
(86) International application number: PCT/EP2020/069657
(87) International publication number: WO 2021/023475

(56) References cited:
- WO-A1-94/13133
- CN-A- 109 258 578
- BHATTARAI UPENDRA RAJ ET AL: "Insights into the Temporal Gene Expression Pattern inLymantria disparLarvae During the Baculovirus Induced Hyperactive Stage", VIROLOGICA SINICA, SPRINGER, DE, vol. 33, no. 4, 25 July 2018 (2018-07-25), pages 345 - 358, XP036589212, ISSN: 1674-0769, [retrieved on 20180725], DOI: 10.1007/S12250-018-0046-X

## Description

### Field and object of the invention

The present invention generally refers to rearing boxes for growing insect larvae from egg to the pupa stage (chrysalis).

An object of the invention is to provide a rearing box for the industrial production of insect pupae, preferably for an automatized production process, that increase production rate as well as the quality and uniformity of the pupae by providing a chamber creating a proper environment for the development of larvae and allowing the easy collection of larvae.

### Background of the invention

There are many types of larval rearing modules, which configuration depends on the species of larvae to be reared and on the purpose of use of the larvae.

A traditional larvae rearing technique, consist of paraffin-coated paper bags containing eggs and a diet for feeding the larvae obtained from these eggs, but these bags had the inconvenience that the thickness of the bags had a decisive influence on their correct operation. Thus, an excessive thickness of the bag represented an excessive exudation of the diet, while an insufficient thickness meant the drying of the diet and, therefore, its deterioration.

Consequently and for a long time, paper cups coated with paraffin containing the diet and larvae were also used. Later, plastic cups were also used for the same use. Regardless of the type of vessel used, this larval rearing system had the disadvantage that the removal of the larvae from the inside of the vessels had to be carried out manually one by one, which means a high amount of labor, as well as an important amount of time Invested.

Therefore, such systems for larval rearing were not commercially practical or economically competitive. In addition, it was extremely difficult to control the humidity inside the breeding vessels, which involved the appearance of fungi and bacteria that disturbed the correct functioning of the breeding system

The Spanish patent ES-2.107.176 T3 (validation of the European patent EP-0.676.918), describes a larval rearing system mainly based on a high-density process and an insect rearing unit from eggs to pupae. Specifically, the insect breeding unit comprises a larval space located under a diet space and above a waste space. The larval space comprises a plurality of surfaces arranged vertically perpendicular to said dietary and waste spaces. These vertical surfaces form adhesion surfaces for the insects. However, this rearing system has the drawbacks that the diet space is subjected for long periods of time to a high humidity, and such exposure to high humidity greatly favors the growth of fungi and nutrients in the diet space, which are subsequently transmitted to insects, causing their contamination and deterioration.

The Spanish patent publication ES-2.232.308 A1 describes a rearing box for breeding larvae, comprising a rearing unit with a plurality of vertical growing walls for the larvae, and a diet tray arranged horizontally closing the upper part of the breeding unit, and a waste tray arranged horizontally, closing the lower part of the breeding unit. The growing walls consist of a rectangular frame holding a net in which the larvae move to find the diet, such as several larvae interfere with each other, so they do not have the same size of niche and therefore they might not grow equally. Since the larvae are grouped at different areas, this makes difficult their collection after the breeding process.

Therefore, conventional insect rearing modules have complicated rearing enclosures, where larvae distribute unevenly on supports containing diet or food, wherein the volume of the rearing enclosure is not optimized to achieve maximum larval density in the enclosure, because the larvae are in continuous movement from one side to the other of the feeding surface, thus, the distribution of the larvae inside the rearing enclosure is not controlled. This means that not all the larvae would be fed properly because access to the food is not equal for all, thus, uniformity and quality of the production is not as desired, and many pupae have to be discharged during the final quality control.

On the other hand, it is known the use larvae as living biofactories for the expression of recombinant proteins. For example, the PCT publication WO 2017/046415 describes means and methods to optimize the industrial production of recombinant proteins in insect pupae.

Nevertheless, there is an increasing need for mass production of high quality homogeneous insect pupae for industrial purposes.

### Summary of the invention

The invention is defined in the attached independent claim, and satisfactorily solves the drawbacks of the prior art, by providing an insect rearing box that allows large pupae production of high and uniform quality, specially configured for its use as part of an automatized pupae production process.

The box comprises lateral walls and top and bottom covers attachable to the lateral walls to configure together a rearing chamber for the pupae growing. Top and bottom covers are detachably coupled with the lateral walls, for example top and bottom covers are configured such as they are press-fitted to the lateral walls, so that they are detachable from the lateral walls simply by pulling them away, either manually for example for inspection purpose, or by means of a robot arm.

Preferably, the box is a rectangular prism so that the lateral walls and the top and bottom covers are rectangular and generally flat. There are two parallel large lateral walls and two small parallel walls. Top and bottom covers have respectively main internal flat surfaces.

According to the invention, the box incorporates panels with a plurality of bars placed inside the rearing chamber, and transversally arranged with respect to the top and bottom covers. Preferably, the bars are straight and are orthogonally arranged with respect to the top and bottom covers.

Each bar has at least a free end, which is the top end of the bar, or the bottom end of the bar, or both ends of the bar are free ends.

The bars are dimensioned in such a way that their top and bottom ends are adjacent or near respectively to the top and bottom covers main internal flat surfaces. The bars are arranged to ensure that each bar is at a suitably distance from the surrounding bars, for example this can be achieved with a matrix or similar distribution, in order to individualize small groups of larvae.

For the use of the rearing box, insect eggs are placed all over the internal surface of the bottom cover, and the internal surface of the top cover is filled with a diet or food for the larvae. Once the eggs hatch, the larvae would find their way from the bottom cover toward the diet on the top cover, using the bars as climbing pathways. Once a larva reaches the top end of the bar where it is placed, it would remain there feeding itself until it is converted in a pupa. In this way, when the breeding process is completed all the pupae are placed on the same plane, which facilitates subsequent process like desilking or collection.

Unlike rearing modules of the prior art, (in which large number of larvae accumulate in specific areas consuming all food, whereas other areas are less populated), with the rearing box of the invention only a small number of larvae are grouped at different areas of the top cover, thus, it is ensured that there is enough food for all of them during the entire growing process. This in turn, ensures high quality, simultaneous and uniform pupae production, to obtain a pupae production which is homogeneous in size, weight and general characteristics.

Since the bars are uniformly distributed in the entire volume of the rearing chamber, it the larvae are evenly distributed, thereby optimizing the capacity of the rearing chamber. It has been foreseen, that a rearing box can be used for growing up to 200 hundreds larvae in optimal conditions.

Therefore, the bars individualize small groups of larvae, that do not interfere with other neighboring larvae.

Ventilations windows covered by a mesh are provided at least on two opposing walls. The two opposing walls are covered by a mesh (8a,8b) and are essentially parallel to the panels (10)for proper ambient air circulation across the rearing chamber, such as the larvae inside the rearing chamber are evenly exposed to a controlled environment in terms of humidity and temperature, thus avoiding fungi growth.

According to the claimed invention, the bars are embodied as panels such as the box incorporates a set of these panels placed inside the rearing chamber. The panels are parallel to each other and they can be easily mounted and extracted from the box, for example while assembling the box before it use, for disassembling the box for its disposal, or for inspecting the growing pupae.

Each panel has a stem that runs transversally, preferably orthogonally, to the bars joining them at the middle of the panel, or alternatively at the top part of the panel.

The insect rearing box is conceived as a single-use only product, thus, all the box components, namely: lateral walls, top and bottom covers, panels and bars, are made of a suitable plastic material.

In a preferred embodiment, the lateral walls are integrally formed as a unitary body by injection molding a plastic material. This unitary body is generally planar, and the four lateral walls are rectangular and are linearly arranged one after the other, such as a folding or deformation line is formed between each pair of adjacent walls, thereby, the box is assembled by folding the walls 90° by the folding lines and engaging the free sides.

Some of the advantages of the invention are summarized below:
- controlled distribution of the larvae inside the rearing chamber, that optimizes the number of the insects in rearing chamber to increase production rate and their continuous access to the food ensuring their homogeneous and optimal growth,
- larvae inside the rearing chamber are evenly exposed to a controlled environment in terms of humidity and temperature, thereby avoiding fungi growth and in turn increasing pupae quality,
- the rearing box can be handled by robots in an automatized production process,
- the rearing box can be manufactured in large numbers at low cost, and it can be conveniently stored and transported. All parts of the box are stackable before mounting, as well as the rearing boxes are also stackable for better storage.
- the rearing box is a single use device, which implies an important reduction in the labor costs because cleaning and disinfection tasks are avoided.

### Brief description of the drawings

Preferred embodiments of the invention are henceforth described with reference to the accompanying drawings, wherein:
Figure 1.- shows a perspective view of a preferred embodiment according to the invention.
Figure 2.- shows an exploded view of the embodiment of figure 1.

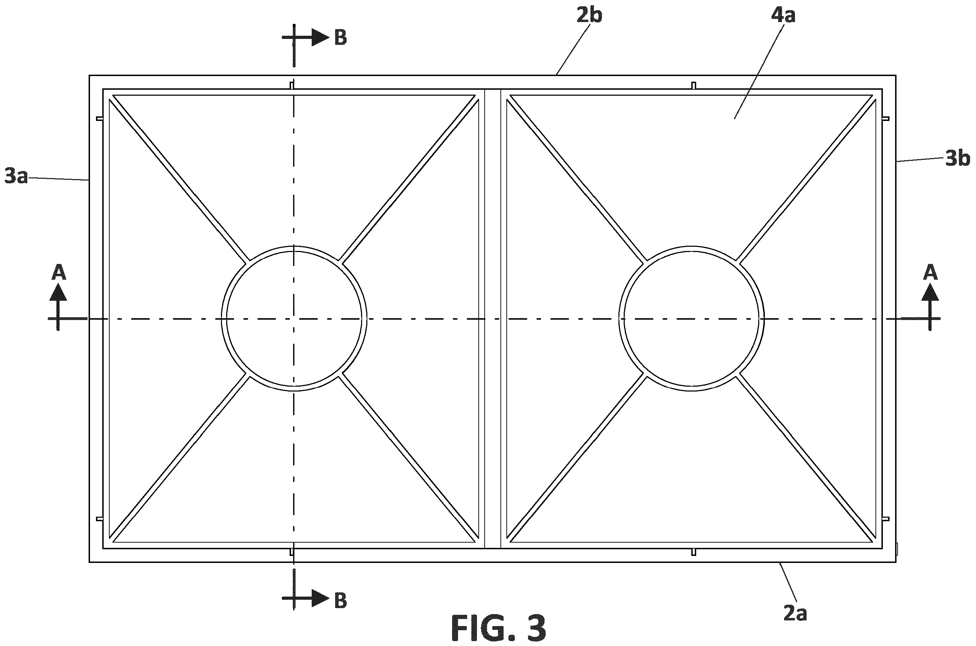

Figure 3.- shows a top plan view of the of the embodiment of figure 1.
Figure 4.- shows an elevational cross-sectional view along plane A-A of figure 3.
Figure 5.- shows a side elevational cross-sectional view along plane B-B of figure 3.
Figure 6.- shows a top plan view with the top cover removed. Arrows on the figure illustrate air circulation inside the rearing chamber.
Figure 7.- shows another perspective view of the embodiment of figure 1, with two side walls and top cover removed.
Figure 8.- shows a plan view of the unitary piece configuring the lateral walls in a deployed state before its assembly.
Figure 9.- shows several views of an alternative configuration of the panel, wherein figure A is a front elevational view, figure B is a top plan view, and figure C is a cross-sectional view taken from plane C-C in figure A.
Figure 10.- shows a similar representation to figure 4 but with the panel of figure 9.

### Preferred embodiment of the invention

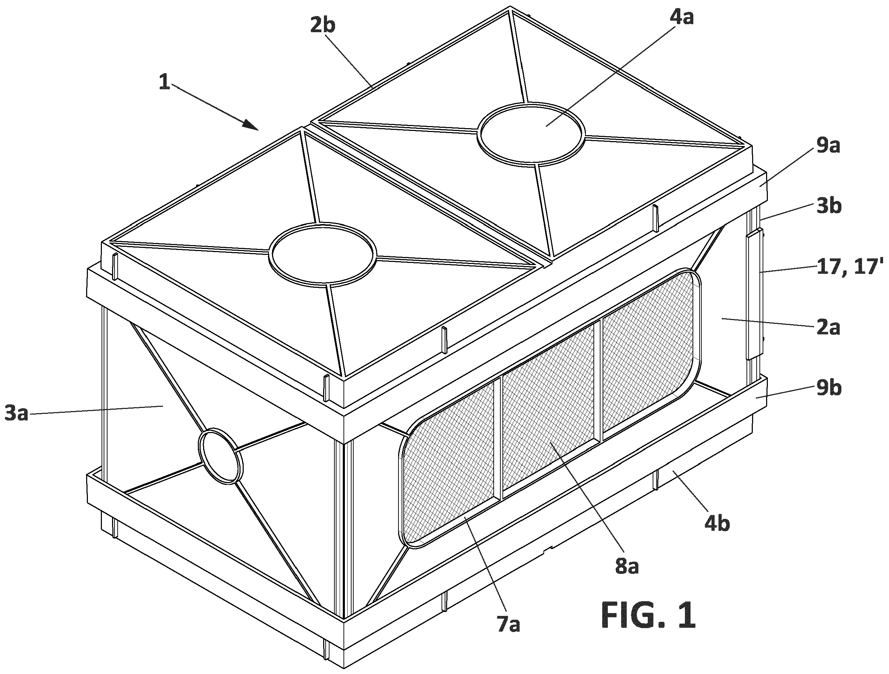

**Figure 1** shows a preferred embodiment of a rearing box (1) according to the invention, comprising: two parallel large lateral walls (2a,2b), two parallel small lateral walls (3a,3b), and top and bottom covers (4a,4b) detachably coupled with the four lateral walls (2a,2b,3a,3b). Top and bottom covers (4a,4b) and the lateral walls (2a,2b,3a,3b), are generally flat, and configure together a rectangular prism, that define internally an insect rearing chamber (6).

The two large lateral walls (2a,2b) have respectively ventilation windows (7a,7b) covered by a mesh (8a,8b), with as a suitable pore size, so the correct light intensity and flow of ventilation air can circulate internally through the rearing chamber (6) for proper pupae growing and, at the same time, avoids the leak of small recently hatched larvae. Alternatively, the small lateral walls (3a,3b) are also provided with ventilation windows (not shown) covered by a similar mesh.

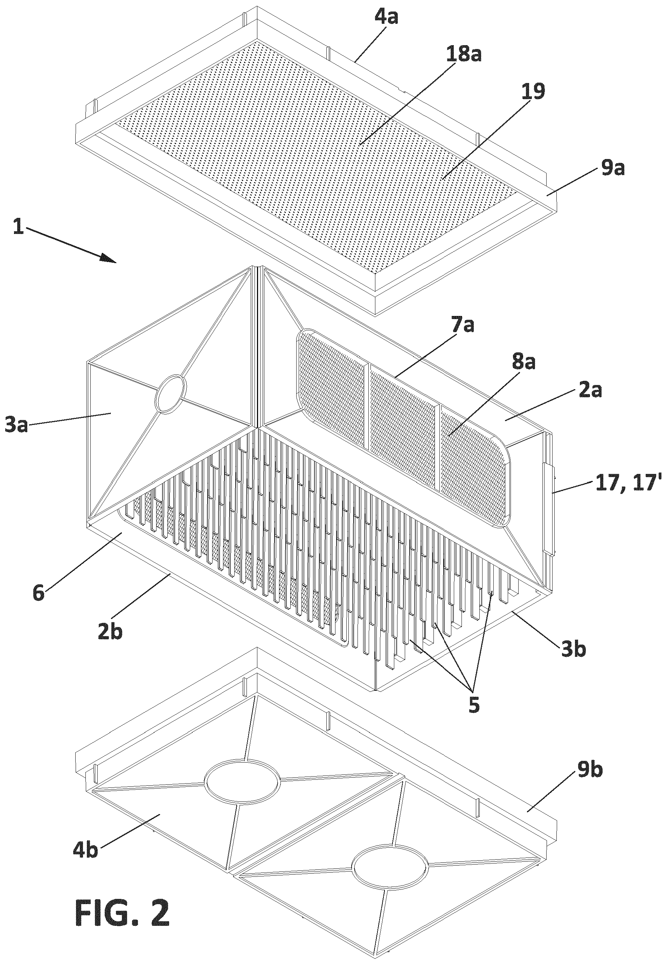

As better shown in **Figure 2****,** top and bottom covers (4a,4b) have the form of trays as they are provided respectively with skirts (9a,9b) extending along the four sides of the covers and protruding transversally from the covers (4a,4b). The top and bottom covers (4a,4b) have respectively main internal flat surfaces (18a,18b). During the use of the box (1), the surface of the top cover (4a) is filled with a diet or food (19) for the larvae, typically in the form of a gel adhered to the surface (18a).

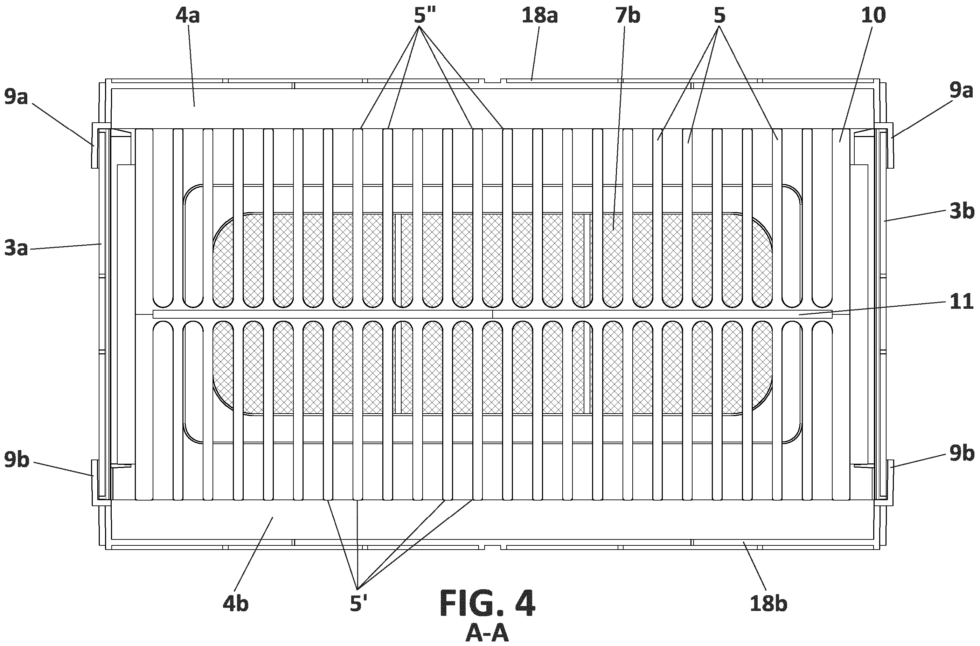

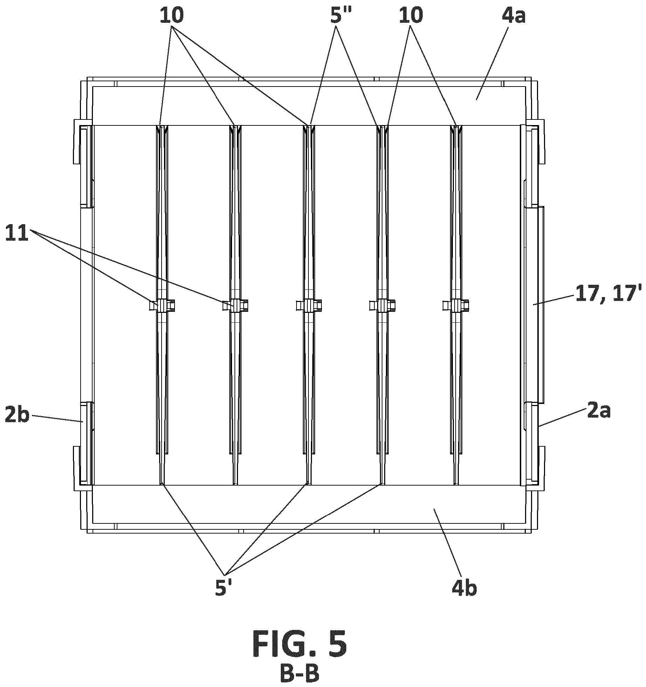

Additionally, top and bottom covers (4a,4b) are configured, that is, they are shaped and dimensioned such as they are press-fitted to the lateral walls (2a,2b,3a,3b) of the box (1) as shown in **Figures 4** **and** **5****,** in such a way that the covers (4a,4b) are detachable from the lateral walls simply by pulling them away. This feature is convenient for removing the covers with a robot, such as to obtain the box without covers (as shown in **Figure 2****),** that is when it is opened, can be submerged in a bath for removing silk from the pupae.

The press-fit feature, is obtained by shaping and dimensioning the interior of the four sides of the skirts (9a,9b), to match the shape and dimension of the outer perimeter of the four lateral walls (2a,2b,3a,3b), as it can be better observed in **Figures 4** and **5****.** The covers (4a,4b) are configured to be stackable in order to save space during storage, so the skirts (9a,9b), also allow a cover to be placed partially inside another cover.

When top and bottom covers (4a,4b) are coupled with the lateral walls (2a,2b,3a,3b), a part of the skirts (9a,9b) is in contact and overlap with the lateral wall, such as the covers (4a,4b) provide rigidity to the box (1) and ensure that each pair of consecutive lateral walls are kept at 90° relative positioning, thus, avoiding deformation of the box during its use.

The box (1) is provided with a plurality of bars (5) placed inside the rearing chamber (6). These bars (5) have top and bottom free ends (5',5") that are adjacent, that is, they are near respectively to the top and bottom covers (4a,4b) main surfaces (18a,18b), such a larva can climb from the bottom cover (4b) and reach food on the internal surface of the top cover from a top end (5") of a bar (5).

The bars (5) are straight, parallel to each other and are orthogonally arranged with respect to the top and bottom covers (4a,4b). Preferably, the bars (5) are flat, and have rough surfaces in order to provide gripping to assist a larva climbing up a bar (5).

According to the claimed invention, the bars (5) are grouped in panels (10) that can be extracted from the box. Therefore, the box (1) incorporates several panels (10) placed inside the rearing chamber (6), such as each panel (10) is planar and configures a plurality of bars (5) parallel to each other and extending orthogonally from a stem (11) as shown for instance in **Figure 4****.** The stem (11) runs transversally to the bars (5) joining them at the middle of the panel (5).

Preferably, the thickness of the bars (5) progressively decreases from the stern (11) towards the free end (5', 5").

As it can be noted for example in **Figure 4****,** each panel has a double-comb configuration. Also in **Figure 4****,** it is noted that all the bars have the same length, and that the all the top ends (5') and the bottom ends are coplanar, and the bottom ends are also coplanar. The box and bars are dimensioned, such as the distance between the top ends (5") of the bars and the main surface (18a) of the top cover (4a), is suitable for the larvae to reach the food from the top ends (5"). Similarly, the box and bars are dimensioned, such as the distance between the bottom ends (5') of the bars and the main surface (18b) of the bottom cover (4b), is suitable for the larvae to reach the bars.

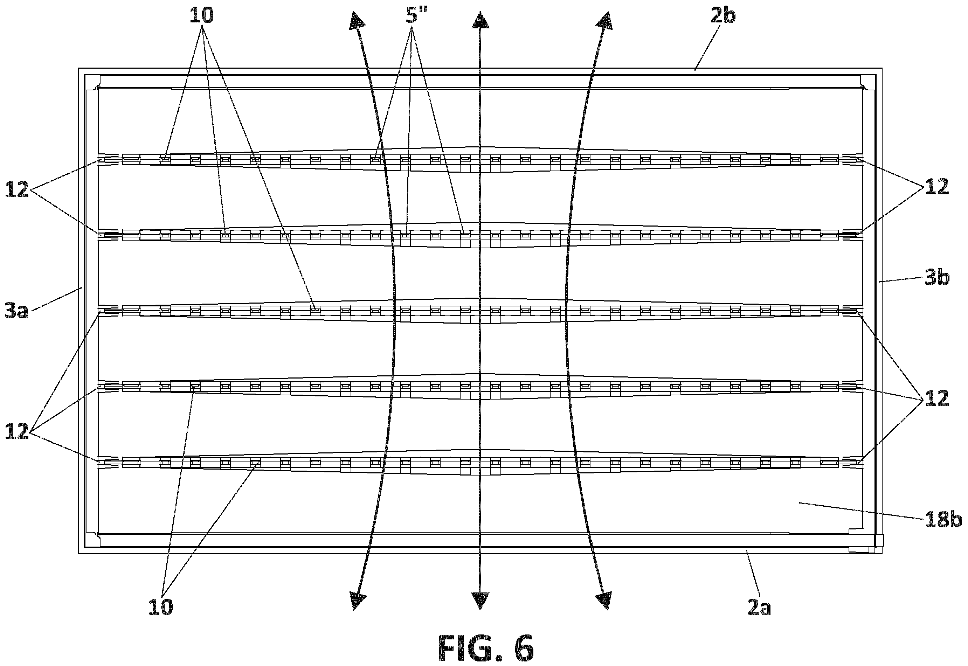

The panels (10) extend lengthwise in the box (1), that is, they are parallel to the large lateral walls (2a,2b) and parallel to the windows (7a,7b). Additionally, and as shown in **Figure 6****,** the bars are arranged defining a matrix in top plant view. With this arrangement, a plurality of channels are defined across the panels (10), that directly communicate with the windows (7a,7b) as shown more clearly in **Figures 4****,** and as indicated by the arrows in **Figure 6****,** thereby enhancing ventilation of the rearing chamber (6).

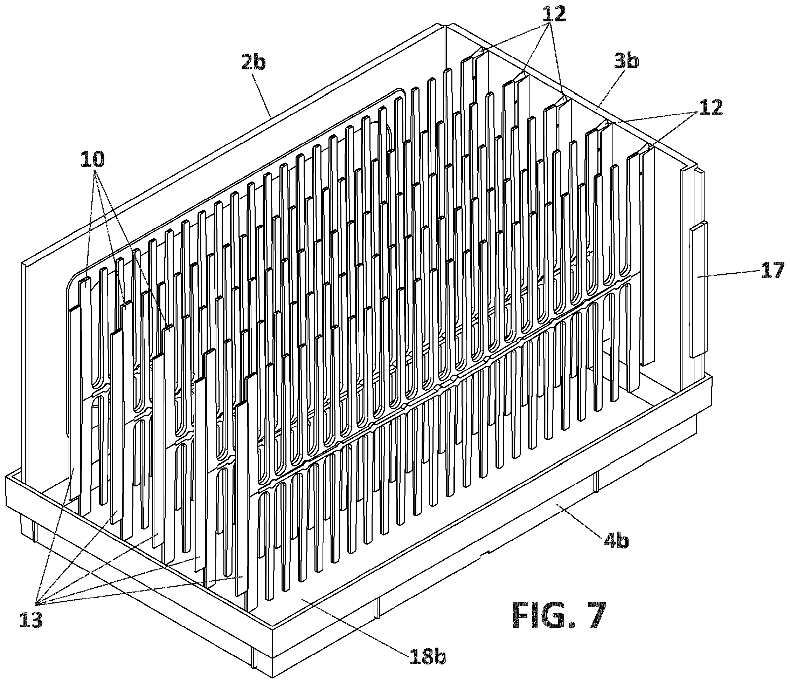

As shown in **Figure 7****,** the two short lateral walls (3a,3b) integrate vertically arranged opposing guides (12), and each panel (10) has tongues (13) at their ends, such as the tongues (13) of each panel can slide vertically on respective guides (12) in which they are inserted. In this way, the panels (10) can be easily assembled in the box (1) in predefined positions or extracted for example for the pupae inspection.

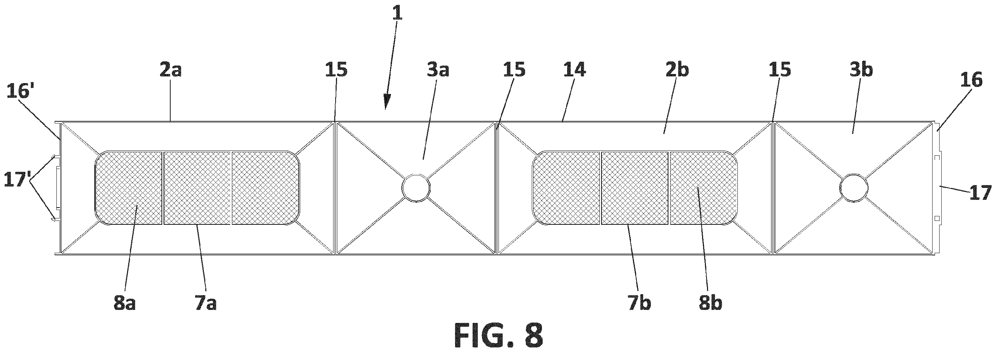

As per the box manufacturing process, the box (1) is made of a suitable plastic material, that is, lateral walls, top and bottom covers and the panels, are made of a plastic material, for example polypropylene. In particular, the lateral walls (2a,2b,3a,3b) are rectangular and are integrally formed as a generally planar and foldable unitary body (14) as shown in **Figure 8****,** by injection molding a plastic material. In the unitary body (14) the walls are linearly arranged, such as each pair of adjacent walls are joined by a folding line (15) which consist of a groove wherein the unitary body is thinner. Preferably, the meshes (8a,8b) are also integrally formed in the unitary body in the same injection molding process.

The construction of the lateral walls as a unitary body (14) as defined above, reduce production costs, and allow that several bodies can be stacked one on top of the other to facilitate storage and transportation in a reduced space.

Additionally, the two free sides (16) of the end walls (2a,3b) in the lineal arrangement are provided with co-operating anchoring means (17,17'), such as for configuring the box (1) the walls (2a,2b,3a,3b) are folded 90° by the folding lines (15) and the cooperating anchoring means (17,17') are engaged as shown for instance in **Figure 2****.** The anchoring means can consist for example in hooks that engage by elastic deformation in openings.

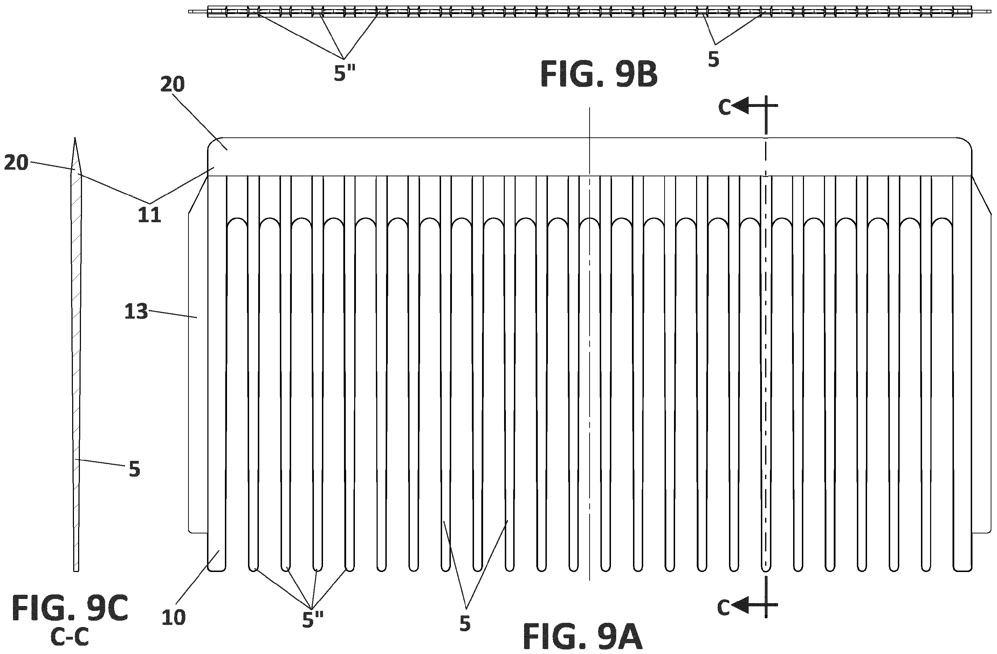

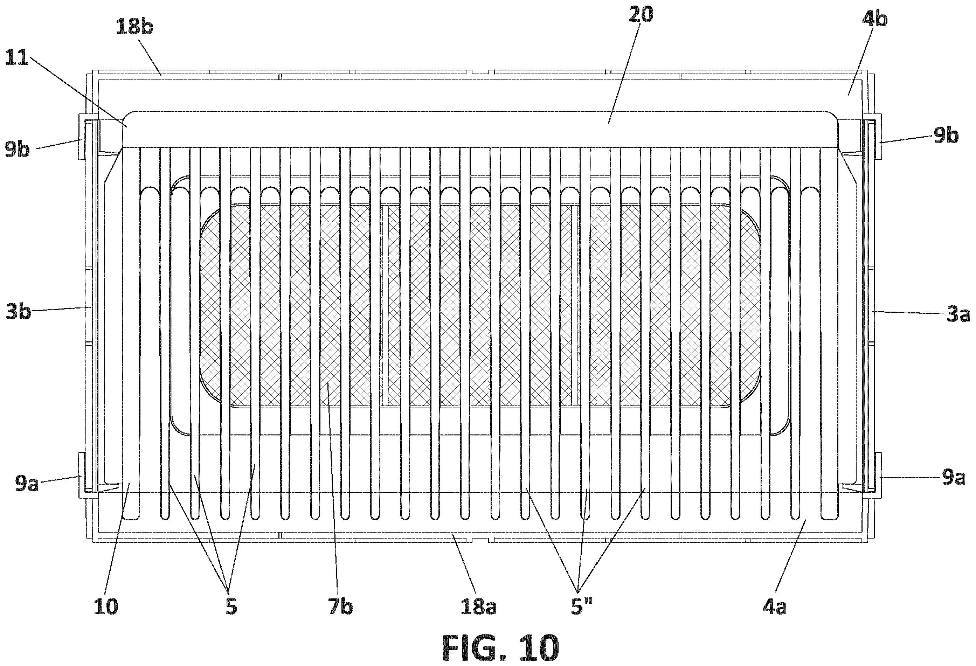

Alternatively in the embodiment of **Figures 9** **and** **10****,** the stem (11) is placed at the top part of the panel and also runs transversally to the bars joining the top ends of the bars, so that only the bottom ends (5") are free ends. In this embodiment the panels are also planar and configures a plurality of bars (5) parallel to each other, with a thickness of the bars (5) progressively decreasing from the stem (11) towards the free bottom end (5'), and also having rough surfaces.

Having the free ends (5") at the lower part of the panel (10) as shown in **Figure 10****,** have the advantage that for the larva is easier to reach the panel and start climbing. Additionally, there is more clearance at the middle area of the box to further enhance ventilation of the rearing chamber (6). Additionally, any debris generated by the larvae is accumulated at the bottom cover instead of a middle area of the panels, far away from the insects. It diminishes potential contaminations of the larvae by fungi. It also helps to clean the rearing boxes, since most of debris are in the bottom cover.

Additionally, in the panel (10) of **Figures 9** and **10** the stem (11) has a sharp edge (20).

## Claims

1. Insect rearing box (1) comprising:
a lateral wall (2a,2b,3a,3b) and top and bottom covers (4a,4b) detachably coupled with the lateral wall to configure a rearing chamber (6),
**characterized in that** the box (1) further comprises two or more panels (10) parallel to each other,
wherein the panels (10) can be extracted from the box and placed inside the rearing chamber (6),
wherein the panels (10) have a plurality of bars (5) placed inside the rearing chamber (6), and transversally arranged with respect to the top and bottom covers (4a,4b),
wherein the bars (5) have top and bottom ends (5',5") adjacent respectively to the top and bottom covers (4a,4b),
wherein the top ends (5') or the bottom ends (5") of the bars (5), are free ends and
wherein the box has two pairs of opposing lateral walls (2a,2b,3a,3b), wherein two opposing walls have ventilations windows (7a,7b) covered by a mesh (8a,8b) and are parallel to the panels (10), and wherein a plurality of channels are defined across the panels (10), that directly communicate with the windows (7a,7b).

2. Insect rearing box (1) according to claim 1, wherein the bars (5) are straight and parallel to each other, and the bars (5) are orthogonally arranged with respect to the top and bottom covers (4a,4b).

3. Insect rearing box according to claim 1 or 2, wherein each panel has generally a planar configuration, and it has a stem (11) and wherein the bars (5) extend orthogonally from the stem (11), and wherein the stem (11) runs transversally to the bars (5) joining them at the middle area between of the panel (5) and top and bottom ends (5',5") of the bars are free ends, or at the top of the panel (10) and the bottom ends (5',5") of the bars are free ends.

4. -Insect rearing box (1) according to any of the preceding claims, wherein the box is a rectangular prism, having two pairs of opposing lateral walls (2a,2b,3a,3b), and wherein the top and bottom covers (4a,4b) are generally flat and parallel to each other.

5. -Insect rearing box (1) according to any of the preceding claims, wherein top and bottom covers (4a,4b) are configured such as they are press-fitted to the lateral walls (2a,2b,3a,3b), such as they are detachable from the lateral walls by pulling them away from the lateral walls.

6. -Insect rearing box (1) according to any of the preceding claims, wherein the lateral walls (2a,2b,3a,3b) are rectangular and are integrally formed as a generally planar and foldable unitary body (14) by injection molding a plastic material, and wherein the walls (2a,2b,3a,3b) are linearly arranged in the unitary body (14) such as each pair of adjacent walls are joined by a folding line (15), and wherein two free sides (16,16') of the end walls in the lineal arrangement are provided with co-operating anchoring means (17,17'), such as by folding the walls 90° by the folding lines (15) the anchoring means (17,17'), can be engaged.

7. Insect rearing box (1) according to claim 6, wherein the meshes (8a,8b) are also integrally formed by in the unitary body (14) in the same injection molding process.

8. Insect rearing box (1) according to claim 3, wherein the stem (11) is placed at the top of the panel (10), and the stem (11) has a sharp edge (20).

9. Insect rearing box (1) according to any of the claims 3 to 8, wherein two opposing walls have vertically arranged guides (12) and each panel (10) has tongues (13) at their ends such as a tongue can slide on a guide, and wherein each panel can be inserted in two opposing guides (13).

10. -Insect rearing box (1) according to any of the preceding claims, wherein the bars (5) are flat, and the bars (5) thickness progressively decreases from the stern (11) towards the free ends (5', 5").

11. -Insect rearing box (1) according to any of the preceding claims, wherein the bars (5) have rough surfaces.

12. -Insect rearing box (1) according to any of the preceding claims, wherein the panels (10) and top and bottom covers (4a,4b) are made of a plastic material.

13. -Insect rearing box (1) according to any of the preceding claims, wherein the bars (5) are arranged as matrix in a top plan view of the rearing chamber (6).

## Patentansprüche

1. Insektenzuchtkasten (1) umfassend:
eine Seitenwand (2a, 2b, 3a, 3b) und eine obere und eine untere Abdeckung (4a, 4b), welche lösbar mit der Seitenwand gekoppelt sind, um eine Zuchtkammer (6) auszubilden,
**dadurch gekennzeichnet, dass** der Kasten (1) zusätzlich zwei oder mehr Paneele (10) umfasst, welche parallel zueinander sind,
wobei die Paneele (10) aus dem Kasten entnommen und innerhalb der Zuchtkammer (6) platziert werden können,
wobei die Paneele (10) eine Vielzahl von Stäben (5) aufweisen, welche innerhalb der Zuchtkammer (6) platziert sind und quer in Bezug auf die obere und die untere Abdeckung (4a, 4b) angeordnet sind,
wobei die Stäbe (5) obere und untere Enden (5', 5") aufweisen, welche jeweils der oberen und der unteren Abdeckung (4a, 4b) benachbart sind,
wobei die oberen Enden (5') oder die unteren Enden (5") der Stäbe (5) freie Enden sind und
wobei der Kasten zwei Paare von gegenüberliegenden Seitenwänden (2a, 2b, 3a, 3b) aufweist, wobei zwei gegenüberliegende Wände Lüftungsfenster (7a, 7b) aufweisen, welche mittels eines Gitters (8a, 8b) bedeckt sind und zu den Paneelen (10) parallel sind, und wobei eine Vielzahl von Kanälen quer durch die Paneele (10) definiert werden, welche mit den Fenstern (7a, 7b) direkt in Verbindung stehen.

2. Insektenzuchtkasten (1) nach Anspruch 1, wobei die Stäbe (5) gerade und parallel zueinander sind, und die Stäbe (5) orthogonal in Bezug auf die obere und die untere Abdeckung (4a, 4b) angeordnet sind.

3. Insektenzuchtkasten nach Anspruch 1 oder 2, wobei jedes Paneel im Allgemeinen eine ebene Ausbildung aufweist und es einen Schaft (11) aufweist, und wobei sich die Stäbe (5) orthogonal vom Schaft (11) aus erstrecken, und wobei der Schaft (11) quer zu den Stäben (5) verläuft, sodass er sie im mittleren Bereich zwischen dem Paneel (5) und den oberen und unteren Enden (5', 5") der Stäbe, welche freie Enden sind, oder im oberen Teil des Paneels (10) und den unteren Enden (5', 5") der Stäbe, welche freie Enden sind, verbindet.

4. Insektenzuchtkasten (1) nach einem der vorhergehenden Ansprüche, wobei der Kasten ein rechteckiges Prisma ist, aufweisend zwei Paare von gegenüberliegenden Seitenwänden (2a, 2b, 3a, 3b), und wobei die obere und die untere Abdeckung (4a, 4b) im Allgemeinen flach und parallel zueinander sind.

5. Insektenzuchtkasten (1) nach einem der vorhergehenden Ansprüche, wobei die obere und die untere Abdeckung (4a, 4b) derart ausgebildet sind, dass sie gegen die Seitenwände (2a, 2b, 3a, 3b) eingepresst werden, sodass sie von den Seitenwänden lösbar sind, indem sie von den Seitenwänden weggezogen werden.

6. Insektenzuchtkasten (1) nach einem der vorhergehenden Ansprüche, wobei die Seitenwände (2a, 2b, 3a, 3b) rechteckig sind und als ein im Allgemeinen ebener und faltbarer einheitlicher Körper (14) mittels Spritzgießens eines Kunststoffmaterials ganzheitlich gebildet sind, und wobei die Wände (2a, 2b, 3a, 3b) im einheitlichen Körper (14) geradlinig angeordnet sind, sodass jedes Paar von benachbarten Wänden mittels einer Faltlinie (15) verbunden sind, und wobei zwei freie Seiten (16, 16') der Endwände in der linearen Anordnung mit zusammenwirkenden Verankerungsmitteln (17, 17') versehen sind, sodass, wenn die Wände 90° um die Faltlinien (15) gefaltet werden, die Verankerungsmittel (17, 17') eingerastet werden können.

7. Insektenzuchtkasten (1) nach Anspruch 6, wobei die Gitter (8a, 8b) auch im einheitlichen Körper (14) im gleichen Spritzgussprozess ganzheitlich gebildet werden.

8. Insektenzuchtkasten (1) nach Anspruch 3, wobei der Schaft (11) im oberen Teil des Paneels (10) platziert ist und der Schaft (11) einen scharfen Rand (20) aufweist.

9. Insektenzuchtkasten (1) nach einem der Ansprüche 3 bis 8, wobei zwei gegenüberliegende Wände vertikal angeordnete Führungen (12) aufweisen und jedes Paneel (10) Zungen (13) an dessen Enden aufweist, sodass eine Zunge auf einer Führung gleiten kann, und wobei jedes Paneel in zwei gegenüberliegende Führungen (13) eingesetzt werden kann.

10. Insektenzuchtkasten (1) nach einem der vorhergehenden Ansprüche, wobei die Stäbe (5) flach sind und sich die Dicke der Stäbe (5) zunehmend vom Schaft (11) zu den freien Enden (5', 5") hin verringert.

11. Insektenzuchtkasten (1) nach einem der vorhergehenden Ansprüche, wobei die Stäbe (5) raue Oberflächen aufweisen.

12. Insektenzuchtkasten (1) nach einem der vorhergehenden Ansprüche, wobei die Paneele (10) und die obere und die untere Abdeckung (4a, 4b) aus einem Kunststoffmaterial hergestellt sind.

13. Insektenzuchtkasten (1) nach einem der vorhergehenden Ansprüche, wobei die Stäbe (5) als Matrix in einer Draufsicht der Zuchtkammer (6) angeordnet sind.

## Revendications

1. Boîte d'élevage d'insectes (1) comprenant :
une paroi latérale (2a, 2b, 3a, 3b) et des couvercles supérieur et inférieur (4a, 4b) accouplés de manière détachable avec la paroi latérale pour configurer une chambre d'élevage (6),
**caractérisée en ce que** la boîte (1) comprend en outre deux ou plusieurs panneaux (10) parallèles les uns aux autres,
dans laquelle les panneaux (10) peuvent être extraits de la boîte et placés à l'intérieur de la chambre d'élevage (6),
dans laquelle les panneaux (10) ont une pluralité de barres (5) placées à l'intérieur de la chambre d'élevage (6), et disposées transversalement par rapport aux couvercles supérieur et inférieur (4a, 4b),
dans laquelle les barres (5) ont des extrémités supérieure et inférieure (5', 5") adjacentes respectivement aux couvercles supérieur et inférieur (4a, 4b),
dans laquelle les extrémités supérieures (5') ou les extrémités inférieures (5") des barres (5) sont des extrémités libres, et
dans laquelle la boîte a deux paires de parois latérales opposées (2a, 2b, 3a, 3b), dans laquelle deux parois opposées ont des fenêtres de ventilation (7a, 7b) recouvertes par un filet (8a, 8b) et sont parallèles aux panneaux (10), et dans laquelle une pluralité de canaux sont définis à travers les panneaux (10), qui communiquent directement avec les fenêtres (7a, 7b).

2. Boîte d'élevage d'insectes (1) selon la revendication 1, dans laquelle les barres (5) sont droites et parallèles les unes aux autres, et les barres (5) sont disposées orthogonalement par rapport aux couvercles supérieur et inférieur (4a, 4b).

3. Boîte d'élevage d'insectes selon la revendication 1 ou 2, dans laquelle chaque panneau a généralement une configuration plane, et il a une tige (11) et dans laquelle les barres (5) s'étendent orthogonalement depuis la tige (11), et dans laquelle la tige (11) s'étend transversalement aux barres (5) les rejoignant dans la zone centrale du panneau (5) et les extrémités supérieure et inférieure (5', 5") des barres sont des extrémités libres, ou dans la partie supérieure du panneau (10) et les extrémités inférieures (5', 5") des barres sont des extrémités libres.

4. Boîte d'élevage d'insectes (1) selon l'une quelconque des revendications précédentes, dans laquelle la boîte est un prisme rectangulaire, ayant deux paires de parois latérales opposées (2a, 2b, 3a, 3b), et dans laquelle les couvercles supérieur et inférieur (4a, 4b) sont généralement plats et parallèles l'un à l'autre.

5. Boîte d'élevage d'insectes (1) selon l'une quelconque des revendications précédentes, dans laquelle les couvercles supérieur et inférieur (4a, 4b) sont configurés de sorte qu'ils sont emboîtés dans les parois latérales (2a, 2b, 3a, 3b), de sorte qu'ils peuvent être détachés des parois latérales en les séparant des parois latérales par traction.

6. Boîte d'élevage d'insectes (1) selon l'une quelconque des revendications précédentes, dans laquelle les parois latérales (2a, 2b, 3a, 3b) sont rectangulaires et sont formés intégralement comme un corps unitaire généralement plat et pliable (14) par le biais de moulage par injection d'un matériau plastique, et dans laquelle les parois (2a, 2b, 3a, 3b) sont disposées linéairement dans le corps unitaire (14) de sorte que chaque paire de parois adjacentes sont reliées par une ligne de pliage (15), et dans laquelle deux côtés libres (16, 16') des parois d'extrémité dans la disposition linéaire sont pourvues de moyens d'ancrage coopérants (17, 17'), de sorte qu'en pliant les parois 90° par les lignes de pliage (15) les moyens d'ancrage (17, 17') peuvent être engagés.

7. Boîte d'élevage d'insectes (1) selon la revendication 6, dans laquelle les filets (8a, 8b) sont également formés intégralement par le corps unitaire (14) dans le même procédé de moulage par injection.

8. Boîte d'élevage d'insectes (1) selon la revendication 3, dans laquelle la tige (11) est placée dans la partie supérieure du panneau (10), et la tige (11) a un bord tranchant (20).

9. Boîte d'élevage d'insectes (1) selon l'une quelconque des revendications 3 à 8, dans laquelle deux parois opposées ont des guides disposées verticalement (12) et chaque panneau (10) a des languettes (13) dans ses extrémités de sorte qu'une languette peut glisser sur un guide, et dans laquelle chaque panneau peut être inséré dans deux guides opposés (13).

10. Boîte d'élevage d'insectes (1) selon l'une quelconque des revendications précédentes, dans laquelle les barres (5) sont plates, et l'épaisseur des barres (5) se réduit progressivement depuis la tige (11) vers les extrémités libres (5', 5").

11. Boîte d'élevage d'insectes (1) selon l'une quelconque des revendications précédentes, dans laquelle les barres (5) ont des surfaces rugueuses.

12. Boîte d'élevage d'insectes (1) selon l'une quelconque des revendications précédentes, dans laquelle les panneaux (10) et les couvercles supérieur et inférieur (4a, 4b) sont fait en un matériau plastique.

13. Boîte d'élevage d'insectes (1) selon l'une quelconque des revendications précédentes, dans laquelle les barres (5) sont disposées comme une matrice dans une vue en plan supérieure de la chambre d'élevage (6).
